# EUROPÄISCHE PATENTSCHRIFT

(11) **EP 0 511 451 B1**
(45) Veröffentlichungstag und Bekanntmachung des Hinweises auf die Patenterteilung: **17.01.1996**
(21) Anmeldenummer: 91810337.5
(22) Anmeldetag: 01.05.1991
(51) Int. Cl.: A61K 7/02

(54) **Form-, Modellier- und Abdeckmasse für Maskenbildnerei und Kosmetik**
Moulding, modelling and covering material for mask-sculpturing and cosmetic use
Matériau de couverture, de modellage et de moulage pour la sculpture de masques et pour une utilisation cosmétique

(43) Veröffentlichungstag der Anmeldung: 04.11.1992
(73) Patentinhaber: Balmer, René, CH-3006 Bern (CH)
(72) Erfinder: Balmer, René, CH-3006 Bern (CH)
(74) Vertreter: Fischer, Franz Joseph

(56) Entgegenhaltungen:
- EP-A- 0 021 135
- FR-A- 1 198 679
- US-A- 2 124 767
- PATENT ABSTRACTS OF JAPAN, Band 10, Nr. 350 (C- 387)(2406), 26. November 1986; & JP-A-61152236 (MEIJI MILK PROD) 10.07.1986

## Beschreibung

Gegenstand der vorliegenden Erfindung ist eine Masse für Maskenbildnerei und Kosmetik, mit welcher Hautveränderungen sowie Gesichts- und Körperplastiken hergestellt werden können. Die erfindungsgemässe Masse auf Basis von Glycerin und Gelatine kann aus natürlichen Ingredienzien hergestellt werden, welche keine gesundheitsschädliche Wirkung zeigen.

In der Maskenbildnerei wurden bisher für die Durchführung von körperplastischen Veränderungen vorallem Materialien aus Silikon verwendet. Diese Substanzen haben den Nachteil, dass sie teuer, unangenehm zu tragen und möglicherweise giftig sind. Zur Entfernung solcher Massen sind organische Lösungsmittel, wie Alkohol und Benzin erforderlich, welche die Haut angreifen.

In der US-124 767 ist ein Maskenbildungsmaterial beschrieben, das unter anderem Gelatine und als Plastifizierungsmittel Glycerin oder ähnliche Substanzen enthält. Dabei ist der Glycerinanteil ungefähr doppelt so gross wie der Gelatineanteil. Zur Befestigung der Masse auf dem Gesicht eines Schauspielers ist ein Klebstoff erforderlich und in der offenbarten Zusammensetzung wird als Ausfällungsmittel der Masse Formaldehyd zugesetzt. Im Gegensatz dazu braucht aufgrund des Verhältnisses von Gelatine zu Glycerin in der vorliegenden Erfindung kein Klebstoff angewandt zu werden und ebenfalls ist die Zugabe des gesundheitsschädlichen Formaldehyds überflüssig.

Es ist demzufolge Aufgabe der vorliegenden Erfindung, ein Material für die Maskenbildnerei und die Kosmetik zur Verfügung zu stellen, welches kostengünstig, ungiftig, gut modellier- und formbar und leicht zu entfernen ist. Eine Aufgabe besteht auch darin, ein Material zugänglich zu machen, das mit einer Verdünnungslösung, die vorwiegend aus Wasser besteht, verdünnbar ist. Eine weitere Aufgabe besteht in der Bereitstellung eines modular aufgebauten Produktes, das durch zweckmässiges Mischen von Grundmassen mit Additiven und/oder Verdünnungsmittel die Herstellung von Massen für unterschiedliche Anwendungen erlaubt. Ein Ziel besteht ebenfalls darin, eine Masse zur Verfügung zu stellen, die ohne die Applikation einer Deckschicht angewandt werden kann.

Diese Aufgaben werden durch die vorliegende Erfindung gelöst. Sie hat eine Form-, Modellier- und Abdeckmasse für Maskenbildnerei und Kosmetik, die durch einen Gehalt von Glycerin und Gelatine mit ungefähr gleichen Anteilen bezogen auf das Gewicht gekennzeichnet ist, zum Gegenstand. Die in Form einer Emulsion vorliegende erfindungsgemässe Masse, die in der Regel, bezogen auf das Glycerin, einen Gelatine-Überschuss aufweist, besitzt, je nach Zusammensetzung und Temperatur, eine dünn- bis dickflüssige und gegebenenfalls eine gummiartige Konsistenz. Das Gewichtsverhältnis von Gelatine zu Glycerin beträgt etwa 1 : 1. Die Masse dient der Maskenbildnerei in Theater, Film und Fernsehen. In speziellen Fällen kann sie jedoch ebenfalls in der Kosmetik angewandt werden, wenn es darum geht, eine Narbe oder eine ähnliche Stelle abzudecken. Die plastische Masse erlaubt es dem Fachmann, in einem Schnellverfahren auf einfache Weise oder aber in anspruchsvoller Weise Formen am Gesicht oder an anderen Körperteilen aufzutragen. Die Masse kann ohne spezielle Kleber aufgetragen werden und kann durch spezielle Farbkonzentrate vorgefärbt werden, wodurch eine Schmink- bzw. Ueberschminktechnik überflüssig wird. Das Material kann auf solche Weise pigmentiert werden, dass es ohne zusätzliche Behandlung hautartig wirkt.

Obschon sich die vorliegende Erfindung auf Massen bezieht, denen auch künstliche Additive zugesetzt werden können, ist die Masse in ihren bevorzugten Ausführungsformen vorwiegend aus natürlichen Substanzen zusammengesetzt, die in der Regel weder toxisch noch allergisch oder sonst gesundheitsschädigend wirken. Dadurch werden mögliche Allergien auf synthetische Konservierungsmittel bzw. Emulgatoren weitgehend vermieden. Beispiele von Farbstoffen, die zugesetzt werden können, sind: Chinolingelb (E 104), Ponceau 4R (E 124), Patentblau VCA (E 131), Brillantschwarz (E 151) und Kaolin (weisser Ton). Um dem Produkt einen angenehmen Geruch zu verleihen, wird es parfümiert. Vorzugsweise wird hierzu Rosenwasser verwendet.

Durch die Auswahl von geeigneten Additiven können die Eigenschaften des Produktes wunschgemäss modifiziert werden. Die Zugabe von Alaun (KAl(SO₄)₂) erhöht die Bindung innerhalb der Masse und besitzt eine stabilisierende und zusammenziehende Wirkung. Der Zusatz von Ricinusöl verleiht eine fleischartige Konsistenz. Der Zusatz von Harnstoff führt zu einem verbesserten Rückhalt der Feuchtigkeit. Weizenstärke hat die Funktion eines Verdickers, Kaugummigrundmasse führt zu einer Verfestigung und Karayae verursacht eine Hautbildung auf der verarbeiteten Masse.

Als Konservierungsmittel wird normalerweise Meproester 25 % (Siegfried AG CH-4800 Zofingen) eingesetzt. Als Emulgatoren können Ester von Diolen mit Fettsäuren verwendet werden, wie z.B. Tegin ^{(R)}, welches aus Estern von Ethylenglykol oder von 1,2-Propandiol mit natürlichen Fettsäuren zusammengesetzt ist.

Die erfindungsgemässen Massen können in Form von haut-, faszien-, knorpel-, sehnen- oder schleimhautartigen Massen vorliegenden, die weich bis zähelastisch hart sein können. In der Regel werden diese Massen durch Erwärmen auf eine Temperatur von 40 bis 50°C verarbeitungsfähig gemacht. Sie besitzen ein eigenes Haftvermögen, so dass keine zusätzlichen Haftmittel zum Einsatz gelangen müssen. Die Entfernung der plastischen Modelliermasse erfolgt durch Abziehen der Plastik und Reinigen mit warmem Wasser. Das hautstrapazierende Reinigen mit organischen Lösungsmitteln, wie Alkohol oder Benzin, fällt weg.

Die Modelliermasse kann durch eine wässrige Lösung, die ca. 1 % Alkohol enthält, verdünnt werden. Dadurch kann der Masse eine solche Konsistenz verliehen werden, wie sie für die jeweilige Verarbeitung erforderlich ist.

Bestimmte Rezepturen der plastischen Masse können ebenfalls geschäumt werden. Hierzu gelangt ein Schäummittel zur Anwendung, wodurch Füllungen möglich sind, welche dicken Aufträgen dienen. Ein solches Schäummittel kann ein Pulvergemisch sein, das in feuchtem Zustand ein Gas freisetzt. In Frage kommt z.B. eine Mischung von peroxidhaltigem Pulver (z.B. Natrumperborat) mit Natriumcarbonat.

Zur Abtönung der plastischen Masse dienen Farbkonzentrate in flüssiger Form, z.B. in den Farben Rot, Blau, Gelb und Schwarz. Dadurch wird eine transparente und hautechte Teinture bewirkt, die dadurch zustandekommt, dass die Farbpigmente nicht auf sondern, in der plastischen Masse abgelagert wird.

Als weisse Farbe wird Kaolinpulver verwendet, welches als solches der flüssigen bzw. aufgeweichten, plastischen Masse zugemischt wird.

Die erfindungsgemässe plastische Masse für die Maskenbildnerei und die Kosmetik kann in verschiedenen Rezepturen auf den Markt gebracht werden. Zur plastischen Masse gehört ebenfalls eine Verdünnungslösung, ein Schäummittel und Farbkonzentrate.

Die vorliegende Erfindung wird anhand der nachstehenden Beispiele, welche spezielle Ausführungsformen darstellen, näher erläutert.

Die angegebenen Bestandteile werden in Reinheitsgraden eingesetzt, wie sie zur Herstellung von kosmetischen oder dermatologischen Präparaten üblich sind. Wenn nicht anders angegeben, wird zur Herstellung der Massen in den nachstehenden Beispielen ausschliesslich destilliertes Wasser eingesetzt.

### Beispiel 1 (Dermplast I)

Aus den Komponenten der nachstehenden Tabelle wird eine Mischung hergestellt:

**Tabelle I**

| Produkte | Lösung | Menge |
|---|---|---|
| Aqua-Dest | - | 20 ml |
| Glycerin | 85 % | 19 ml |
| Gelatine-Pulver | - | 21 g |
| Alaun (gelöst in Aqua-Dest) | 6 ‰ | 5 ml |
| Meproester | 25 % | 1 ccm |
| Parfum (Rosenwasser) | - | 3,5 ccm |
| Farbstoff E | | |

Das Wasser und das Glycerin werden in ein Gefäss gegeben und anschliessend wird das Gelatinepulver mit dieser Flüssigkeit angerührt und auf eine Temperatur von 80 bis 90°C erwärmt. Das Mischen wird fortgesetzt, bis eine homogene Masse entsteht, dann wird der Alaun eingerührt und das Wasser bis zu 80 % verdampft. Schliesslich wird der erhaltenen Masse als Konservierungsmittel der Meproester zugesetzt, mit Rosenwasser parfümiert und mit einer Farblösung oder einem Farbpulver gefärbt.

Es wird eine zähelastische Masse erhalten, die nach der Anwendung hornhautartig aussieht und einen Schrumpfungseffekt besitzt. Die Farbe ist transparent gelbgrünlich.

Die Masse kann als Elastizitätsverstärker für die Produkte gemäss den Beispielen 2 und 4 verwendet werden. Ebenso ist eine direkte Verarbeitung an Gesichts- und Körperteilen, wie auch eine indirekte Verarbeitung in Negativ- und Positivformen möglich. Dabei kann das Material gespritzt, gegossen, gestrichen oder geschäumt werden, wobei die Verarbeitungskonsistenz der Applikationsweise anzupassen ist.

### Anwendung:

Die plastische Masse wird in einem Glas- oder Keramikgefäss auf eine Temperatur von 45 bis 50°C erwärmt. Dabei findet eine Verflüssigung statt. Zur Erzielung der gewünschten Konsistenz wird Verdünnungslösung (Wasser mit ca. 1 % Alkohol) zugegeben. Es können 1 bis 100 Gew.-% des Verdünnungsmittels zugesetzt werden. Ist die Viskosität des Materials zu hoch, lässt sich das Material nur unbefriedigend verarbeiten.

Je nach dem Gehalt des Verdünnungsmittels beträgt die Aushärtungszeit 30 sec bis mehrere h. Die Aushärtung findet unter Abkühlung und Verflüchtigung des Verdünnungsmittels statt. Durch Erwärmung kann die Aushärtungszeit wesentlich verkürzt werden. Es ist eine Temperatur bis 35°C möglich. Als Arbeitsmittel kommen dabei Föhn, Trockenkammer oder Trockenhaube in Frage. Dabei ist zu beachten, dass ein plastisches Material für die direkte Applikation auf der Haut eine kürzere Aushärtungszeit besitzen muss, als in einer Negativ- bzw. Positivform.

### Beispiel 2 (Dermplast II)

Die Masse setzt sich aus folgenden Komponenten zusammen:

| Produkte | Lösung | Testmenge |
|---|---|---|
| Aqua-Dest | - | 20 ml |
| Glycerin | 85 % | 20 ml |
| Gelatine-Pulver | - | 21 g |
| Tegin (versprüht) | - | 1,5 ccm |
| Ricinus-Oel | - | 15 ccm |
| Alaun (gelöst in Aqua-Dest) | 6 ‰ | 5 ml |
| Harnstoff (gelöst in Aqua-Dest) | 2 % | 10 ccm |
| Meproester | 25 % | 1,5 ccm |
| Parfüm (Rosenwasser) | - | 1,5 ccm |
| Farbstoff E | | |

Das destillierte Wasser und das Glycerin werden in ein Gefäss gegeben und mit der erhaltenen Flüssigkeit wird das Gelatinepulver angerührt. Anschliessend wird Tegin^{(R)}, Ricinus-Oel, Alaun und Harnstoff in das Gemisch eingerührt und auf eine Temperatur von 80 bis 90°C erwärmt und gerührt bzw. gemischt, bis eine homogene, nicht mehr körnige Masse entsteht. Schliesslich wird der gewünschte Farbstoff beigemischt und anschliessend der Meproester zur Konservierung und das Rosenwasser zur Parfümierung zugefügt.

Es wird eine elastische Masse erhalten, die, wenn aufgetragen, eine faszienartige Eigenschaft besitzt. Sie hat ein transparentes, gleichfarbig helles Aussehen.

Die Masse eignet sich sowohl für die direkte Verarbeitung an Gesichts- oder Körperteile, wie auch für die indirekte Verarbeitung in Negativ- bzw. Positivformen. Als Elastizitätsverstärker kann das gemäss Beispiel 1 hergestellte Produkt zugemischt werden. Im übrigen kann das Produkt in gleicher Weise, wie in Beispiel 1 angegeben, verarbeitet werden.

### Beispiel 3 (Dermplast III)

Es wird eine Masse aus folgenden Komponenten hergestellt:

| Produkte | Lösung | Testmenge |
|---|---|---|
| Aqua-Dest | - | 70 ml |
| Glycerin | 85 % | 20 ml |
| Weizenstärke | - | 60 ccm |
| Gelatine-Pulver | - | 20 g |
| Tegin (versprüht) | - | 3,5 ccm |
| Ricinus-Oel | - | 15 ccm |
| Alaun (gelöst in Aqua-Dest) | 6 ‰ | 5 ml |
| Harnstoff (gelöst in Aqua-Dest) | 2 % | 10 ccm |
| Meproester | 25 % | 1,5 ccm |
| Parfüm (Rosenwasser) | - | 4,5 ccm |
| Farbstoff E | | |

Das destillierte Wasser und das Glycerin werden in ein Gefäss gegeben, wonach Weizenstärke eingerührt wird. Die erhaltene Masse wird mit Gelatinepulver vermischt und anschliessend wird Tegin, Ricinus-Oel, Alaun und Harnstoff eingearbeitet und schnell auf eine Temperatur von 100°C erwärmt, bis die Weizenstärke unter ständigem Rühren vernetzt (gebunden) ist. Ist die Masse homogen, wird die Temperatur auf 80°C reduziert und das destillierte Wasser unter Rühren bis zu etwa 80 % abgedampft. Schliesslich wird der Farbstoff, der Meproester und das Rosenwasser zugegeben. Es wird ein leicht elastisches, knorpelartiges Material erhalten, das ein transparent, gelb-grünlich und mattes Aussehen besitzt. Es kann direkt an Gesichts- oder Körperteilen verarbeitet werden, wobei es gestrichen, gespachtelt oder modelliert werden kann.

Zur Verarbeitung wird das Material in ein Glas- oder Keramikgefäss gegeben und mit einer Alufolie zugedeckt. Anschliessend wird die Masse auf einem Wasserbad auf eine Temperatur von 45 bis 50°C erwärmt. Dabei wird sie klebrig und knetig.

Zum Spachteln und Modellieren wird das Produkt direkt verwendet, wobei kein oder nur tropfenweise Verdünnungsmittel zugesetzt wird. Zum Streichen hingegen wird das Verdünnungsmittel zugegeben und das Material gut durchgespachtelt, bis es in der gewünschten Konsistenz vorliegt. Die Aushärtungszeit des Produktes beträgt 1 bis 2 min in der unverdünnten Form (Spachtel- oder Modellierkonsistenz) und bis zu 15 min in der Streichkonsistenz. Die Aushärtungszeit kann mit Föhn, Trockenkammer oder Trockenhaube reduziert werden.

### Beispiel 4 (Dermplast IV)

Aus den nachstehenden Komponenten wird eine Formmasse hergestellt:

| Produkte | Lösung | Testmenge |
|---|---|---|
| Aqua-Dest | - | 40 ml |
| Glycerin | 85 % | 19 ml |
| Weizenstärke | - | 20 ccm |
| Gelatine-Pulver | - | 21 g |
| Gips | - | 4,5 ccm |
| Kaumasse | - | 20 g |
| Meproester | 25 % | 1,5 ccm |
| Parfüm (Rosenwasser) | - | 3,5 ccm |

Das destillierte Wasser und das Glycerin werden in ein Gefäss gegeben und anschliessend die Weizenstärke gut eingerührt. Anschliessend wird Gelatinepulver und Gips zugegeben und die Mischung unter ständigem Rühren auf ca. 80°C erwärmt, bis ein homogener, milchiger Brei vorliegt. Dann wird Kaumasse (Kaugummigrundmasse) zugegeben, bis eine einheitliche, fadenziehende Masse entsteht. Die Temperatur wird auf 100°C erhöht und das Produkt wird weitergerührt, bis die fadenziehende Masse cremig wird. Anschliessend wird die Temperatur auf 80°C reduziert und dem Produkt bis zu 80 % Wasser durch Verdampfen entzogen. Schliesslich wird Meproester und Rosenwasser als Konservierungsmittel bzw. Duftstoff zugegeben.

Es wird ein wenig dehnkräftiges, hartes und sehnenartiges Produkt mit beige-mattem Aussehen erhalten. Es eignet sich für die direkte Verarbeitung in Gips- oder Silikon-Negativformen, für die direkte Verarbeitung an Gesichts- oder Körperteilen und für die direkte Verarbeitung auf Positivformen, wenn nicht zu grosse Hinterschneidungen vorhanden sind. Das Produkt kann gestrichen oder modelliert werden. Zur Verarbeitung wird das Produkt in einem gedeckten Glas- oder Keramikgefäss auf dem Wasserbad auf eine Temperatur von 45 bis 50°C erwärmt. Für die grossflächige Verarbeitung an Gips- oder Silikonformen darf die Temperatur bis auf etwa 80°C erhöht werden.

Zum Modellieren wird kein oder nur tropfenweise Verdünnungsmittel zum gummiartig weichen Material zugegeben. Zum Streichen muss soviel Verdünnungsmittel zugegeben und durchgeknetet werden, bis die gewünschte Konsistenz vorliegt. Die Aushärtungszeit beträgt 2 min in Modellierkonsistenz und etwa 30 min der Streichkonsistenz. Die Trocknung erfolgt nur mit Kaltluft.

### Beispiel 5 (Dermplast V)

Aus den nachstehenden Komponenten wird eine Masse hergestellt:

| Produkte | Lösung | Testmenge |
|---|---|---|
| Aqua-Dest | - | 40 ml |
| Glycerin | 85 % | 22 ml |
| Weizenstärke | - | 60 ccm |
| Gelatine-Pulver | - | 21 g |
| Tegin (versprüht) | - | 3,5 ccm |
| Ricinus-Oel | - | 15 ccm |
| Karayae (gequollen in Aqua-Dest) | - | 100 ccm |
| Alaun (gelöst in Aqua-Dest) | 6 ‰ | 5 ml |
| Milchsäure | - | 3,5 ccm |
| Meproester | 25 % | 1,5 ccm |
| Parfüm (Rosenwasser) | - | 4,5 ccm |
| Farbstoff E | | |

Das destillierte Wasser und das Glycerin werden in ein Gefäss gegeben und anschliessend wird Weizenstärke eingerührt. Dieser Masse wird Gelatine-Pulver zugemischt. Anschliessend wird Tegin (Warenzeichen), Ricinus-Oel, Karayae, Alaun und Milchsäure zugemischt und auf 100°C solange erwärmt, bis die Weizenstärke unter ständigem Rühren vernetzt (gebunden) ist und eine homogene Masse entsteht. Die Temperatur wird auf 80°C reduziert und das Produkt wird unter Abdampfen des Wassers bis zu ca. 60 % eingedickt. Am Schluss wird der Farbstoff, der Meproester und das Rosenwasser zugegeben. Es wird ein Produkt erhalten, das in verarbeiteter Form weich und leicht elastisch ist, eine Folienhaut besitzt und ein hautfarbig helles Aussehen hat. Die Masse kann für die direkte Verarbeitung auf Gesichts- oder Körperteilen ebenso wie für eine indirekte Verarbeitung in Negativ- bzw. Positivformen eingesetzt werden.

Das Produkt eignet sich zum Streichen. Für die Verarbeitung wird die Masse in einem gedeckten Keramik- oder Glasgefäss auf dem Wasserbad auf eine Temperatur von 45 bis 50°C erwärmt. Dabei wird die Masse cremig, wobei nur Verdünnungsmittel zugegeben ist, wenn die Streichfähigkeit vermindert werden soll. Die Aushärtungszeit beträgt, aufgetragen an Gesichts- oder Körperteilen, ca 5 min und in Gips- oder Silikonformen mehrere h. Die Aushärtungszeit kann durch Erwärmen mit Föhn, Trockenkammer oder Trockenhaube wesentlich verkürzt werden.

### Beispiel 6 (Dermplast VI)

Es wird eine Masse aus folgenden Komponenten hergestellt:

| Produkte | Lösung | Testmenge |
|---|---|---|
| Aqua-Dest | - | 70 ml |
| Glycerin | 85 % | 20 ml |
| Weizenstärke | - | 60 ccm |
| Gelatine-Pulver | - | 21 g |
| Tegin | - | 4,5 ccm |
| Ricinus-Oel | - | 15 cc |
| Alaun (gelöst in Aqua-Dest) | 6 ‰ | 5 ml |
| Milchsäure | - | 3,5 ccm |
| Kochsalz | - | 4,5 ccm |
| Meproester | 25 % | 1 ccm |
| Parfüm (Rosenwasser) | - | 4,5 ccm |
| Farbstoff E | | |

Das destillierte Wasser und das Glycerin werden in ein Gefäss gegeben und anschliessend die Weizenstärke gründlich eingerührt. Dann wird die Masse mit dem Gelatine-Pulver gemischt und anschliessend Tegin, Ricinus-Oel, Alaun, Milchsäure und Kochsalz eingerührt und die Temperatur schnell auf 100°C erhöht. Danach wird die Weizenstärke unter ständigem Rühren vernetzt und es entsteht eine homogene Masse. Die Temperatur wird anschliessend auf 80°C reduziert und die Masse eingedickt, indem der Wasseranteil durch Verdampfen zu 90 % reduziert wird. Schliesslich wird der Farbstoff, der Meproester und das Parfüm zugemischt.

Es wird eine weiche Spachtel- und Modelliermasse erhalten, mit einem hautfarbig hellen Aussehen. Sie eignet sich für die direkte Verarbeitung an Gesichts- und Körperteilen. Das Produkt kann von Hand unter leichtem Erwärmen geknetet und direkt anmodelliert oder angespachtelt werden. Dieses Produkt erfordert keine Aushärtungszeit. Die Widerstandsfähigkeit erhält es durch die sofortige leichte Abkühlung und einer Ruhezeit von ca. 2 min.

Das Material eignet sich zum Giessen, Streichen oder Schäumen. Zur Verarbeitung wird die Masse in ein Glas- oder Keramikgefäss gegeben und auf dem Wasserbad auf eine Temperatur von nicht über 100°C erwärmt. Die Masse, die als solche d.h. ohne Verdünnung, zum Einsatz gelangt, wird flüssig. Die Aushärtungszeit beträgt ca. 30 min. Bei geschlossener Form muss die Abkühlung im Kern abgewartet werden. Durch Abkühlen, ohne Einfrieren, kann die Aushärtungszeit verkürzt werden.

### Beispiel 7

### Durchführung einer Hautstrukturveränderung

Es werden eine oder mehrere dünne Schichten des Produktes gemäss Beispiel 1 (Dermplast I) direkt auf die Haut gestrichen, beispielsweise auf die Augenring-Muskelpartie oder Teile davon. Nach dem Aushärtungsprozess entsteht eine Falten- oder Narbenhaut.

Das Runzligmachen der Haut kann durch deren Spannen oder Springenlassen verstärkt werden. Wird der Modelliermasse ein weisses Teintpulver beigemischt, kann ein milchglasartiger Effekt erzielt werden.

### Beispiel 8

### Herstellung von Gesichts- oder Körperplastiken

Hierzu werden Plastiken aus der Masse gemäss Beispiel 2 (Dermplast II), die aus einer Negativform stammen, direkt auf die Haut aufgetragen. Am Schluss wird die Plastik mit mehreren Hornhautschichten aus Dermplast I gemäss Beispiel 1 versehen. Durch die Weichheit der Masse gemäss Beispiel 2, die direkt an der Haut haftet, wird die Mimik des Spielers optimal auf die Plastik übertragen.

Muss ein plastischer Teil besonders widerstandsfähig sein, z.B. bei einer Moulage (Verletzung), die genäht werden muss, kann die Plastik mit Watte armiert werden. Dies kann dadurch geschehen, dass das noch nicht ausgehärtete, klebrige Material mit Watte in Kontakt gebracht wird, wodurch die Wattenfasern auf dem plastischen Material kleben bleiben.

### Beispiel 9

### Herstellung einer künstlichen Glatze

Zuerst wird ein Gipsform-Positiv hergestellt, welches mit dem Material gemäss Beispiel 5 eingestrichen wird. Anschliessend wird mit warmer Luft bis ca. 60°C das Produkt trocknengelassen. Danach wird mit einem Finger durch eine Druckbewegung die noch warme und daher weiche Masse geglättet. Die Uebergänge an der Stirn-, der Ohren- und Nackenpartie werden mit den Produkten gemäss den Beispielen 2 und 1 realisiert. Nach der Abkühlung wird die künstliche Glatze der Form entnommen.

### Beispiel 10

### Herstellung eines Nasenhöckers

Hierzu wird die Masse gemäss Beispiel 6 solange leicht durchgeknetet, bis sie klebrig wird. Anschliessend wird das Material auf die Nase aufgesetzt und geformt, wobei die Uebergänge mit dem Verdünnungsmittel angepasst werden. Ein möglicher unnatürlicher Glanz wird mit Trockenpuder kaschiert.

### Beispiel 11

### Herstellung von Brandblasen

Es wird die Masse gemäss Beispiel 3 fingerkuppengross an den entsprechenden Stellen aufgetragen und mittels Verdünnungslösung in die Blasenform gedrückt. Schliesslich werden die Plastiken mit Teint rot oder blau gefärbt.

### Weitere Anwendungen

Eine Larvengesicht oder eine feste Maske kann durch die Masse gemäss Beispiel 4 hergestellt werden. Ebenso können abgetrennte Körperteile, wie Finger, eine Hand oder ein Kopf nachgebildet werden, indem das Produkt gemäss Beispiel 8 in eine Form gegossen wird. In diesem recht aufwendigen Verfahren werden grössere Weichteile im Kern geschäumt.

### Beispiel für ein Verdünnungsmittel:

Destilliertes Wasser 1000 ml
Ethanol absolut 10 ml
Rosenwasser 20 ml
Farbstoff nach Wunsch
Die Lösung aus den obigen Komponenten ist ein Verdünnungsmittel für die erfindungsgemässen Massen. Je nach der zugegeben Menge können die Massen, beispielsweise diejenigen gemäss den Beispielen 1 bis 5, der gewünschten Konsistenz entsprechend verarbeitungsfertig gemacht werden. Zusätzlich dient das Verdünnungsmittel ebenfalls als Modellierhilfe für die Massen gemäss den Beispielen 3 und 6. Die mit dem Verdünnungsmittel befeuchteten oder getränkten Werkzeuge, wie Pinsel, Spachtel, Finger ermöglichen eine einwandfreie Oberflächenbehandlung der geformten Plastik und helfen, einen tadellosen Plastikhautüberzug zu realisieren. Dadurch kann auch eine Hautstruktur "gestempelt" werden.

Das erwärmte Verdünnungsmittel kann ebenfalls für die Reinigung der verwendeten Werkzeuge eingesetzt werden. Das Verdünnungsmittel ist ebenfalls nützlich zur Bereitstellung eines Materials mit einer bestimmten Konsistenz. Werden beispielsweise die Massen gemäss den Beispielen 1 und 2 zum Spritzen eingesetzt, muss prozentual mehr Verdünnungsmittel im Gemisch vorliegen, als Masse.

Beim Giessen hingegen muss prozentual mehr Masse gemäss den Beispielen 1 oder 2 als Verdünnungsmittel vorliegen.

Zum Streichen können die Massen gemäss den Beispielen 1, 2, 3, 4 oder 5 eingesetzt werden, wobei das Verhältnis von Masse zu Verdünnungsmittel zugunsten der Masse verschoben sein muss. Für die Massen gemäss den Beispielen 1 und 2 muss für das Schäumen mehr Masse als Verdünnungsmittel vorliegen. Zum Spachteln und Modellieren des Produktes gemäss Beispiel 3 darf nur tropfenweise Verdünnungsmittel zugesetzt werden. Zum Schäumen der erfindungsgemässen Massen, beispielsweise der Massen gemäss den Beispielen 1, 2 und 8, kann das Schäumungsmittel (Mousser) aus folgenden Komponenten eingesetzt werden:
Natriumcarbonat 60 %
Natriumperborat-Pulver 3 %-ig 40 % (z.B. "Neutralizer" (90 % Na₄B₂O₇, 10 % Raurylsulfat) Ann Astaire CH-8505 Winterthur)

### Herstellung von Farbkonzentraten (Teint)

Für ein Farbkonzentrat werden folgende Komponenten miteinander gemischt:

| | |
|---|---|
| Destilliertes Wasser | 1000 ml |
| Farbstoff | 12 ml |
| Rosenwasser | 40 ml. |

Diese Farbkonzentrate sind für den normalen Gebrauch bis zu 20 mal zu verdünnen. Die erfindungsgemässen Massen können mit den obengenannten Farblösungen vor oder nach der Applikation eingefärbt werden. Für die genaue Abstimmung der Hautfarbe wird bevorzugt die Farblösung mittels eines Pinsels oder eines Wattestäbchen aufgetragen. Während eine Nachfärbung bei allen in den Beispielen erwähnten Massen möglich ist, kann die Voreinfärbung bei einzelnen Massen nicht zu befriedigenden Ergebnissen führen. Die Massen nehmen die Farbe auf. Es ist deshalb nicht möglich, dass mit der vorliegenden Färbetechnik eine bereits dunkelgefärbte Masse wieder aufgehellt werden kann. Liegen die Farbe in Pulverform vor, ist nur eine Voreinfärbung möglich. Diese erfolgt durch direktes Einstreuen des Farbstoffes in die flüssige oder aufgeweichte Masse. Als Farbstoffe sind beispielsweise erwähnt Chinolingelb (E 104), Ponceau 4R (E 124), Patentblau VCA (E 131) und Brillantschwarz (E 151). Es sind jedoch noch andere Farbstoffe, wie Lebensmittelfarbstoffe, die keine schädlichen Auswirkungen besitzen, verwendbar. Weiter kann Kaolin als Pigment eingesetzt werden.

## Patentansprüche

1. Form-, Modellier- und Abdeckmasse für Maskenbildnerei und Kosmetik in Form einer dünn- bis dickflüssigen W/O-Emulsion, dadurch gekennzeichnet, dass sie auf Basis von Glycerin und Gelatine aufgebaut ist, wobei die Komponenten Glycerin und Gelatine in ungefähr gleichen Gewichtsteilen enthalten sind.

2. Masse gemäss Anspruch 1, dadurch gekennzeichnet, dass sie eines oder mehrere Additive aus der Gruppe der Farbstoffe, Riechstoffe, Emulgatoren und Konservierungsmittel enthält.

3. Masse gemäss Anspruch 1 oder 2, dadurch gekennzeichnet, dass sie 0,1 bis 2 ‰ Alaun enthält.

4. Masse gemäss einem der Ansprüche 1 bis 3, dadurch gekennzeichnet, dass sie 0,2 bis 2,0 % Meproester als Konservierungsmittel enthält.

5. Masse gemäss einem der Ansprüche 1 bis 4, dadurch gekennzeichnet, dass sie zusätzlich eine oder mehrere der folgenden Komponenten enthält: Ester eines niederen Diols mit einer Fettsäure als Emulgator, Ricinusöl, Harnstoff, Weizenstärke, Gips, Kaugummigrundmasse, Karaya, Milchsäure und Kochsalz.

6. Masse nach Anspruch 5, dadurch gekennzeichnet, dass der Ester des niederen Diols ein Ester des Glycerins, des Ethylenglykols oder des 1,2-Propandiols mit einer natürlichen Fettsäure ist.

7. Masse nach einem der Ansprüche 1 bis 6, dadurch gekennzeichnet, dass sie folgende Komponenten enthält:
| | | |
|---|---|---|
| - Glycerin | 45-50 | Gewichtsteile |
| - Gelatine-Pulver | 50-55 | Gewichtsteile |
| - Alaun | 0,07-0,09 | Gewichtsteile |
| - Meproester | 0,5-0,7 | Gewichtsteile |
sowie nach Bedarf Rosenöl zur Parfümierung und Lebensmittel-Farbstoff.

8. Masse nach Anspruch 7, dadurch gekennzeichnet, dass sie zusätzlich folgende Komponenten enthält:
| | | |
|---|---|---|
| - Ester eines niederen Diols mit einer Fettsäure | 4,5-5,0 | Gewichtsteile |
| - Ricinusöl | 35-40 | Gewichtsteile |
| - Harnstoff | 0,04-0,06 | Gewichtsteile |

9. Masse nach Anspruch 7, dadurch gekennzeichnet, dass sie zusätzlich folgende Komponenten enthält:
| | | |
|---|---|---|
| - Weizenstärke | 140-160 | Gewichtsteile |
| - Ester eines niederen Diols mit einer Fettsäure | 8-10 | Gewichtsteile |
| - Ricinusöl | 34-41 | Gewichtsteile |
| - Harnstoff | 0,04-0,06 | Gewichtsteile |

10. Masse nach einem der Ansprüche 1-6, dadurch gekennzeichnet, dass sie folgende Komponenten enthält:
| | | |
|---|---|---|
| - Glycerin | 45-50 | Gewichtsteile |
| - Weizenstärke | 45-55 | Gewichtsteile |
| - Gelatine-Pulver | 50-55 | Gewichtsteile |
| - Gips | 9-14 | Gewichtsteile |
| - Kaugummigrundmasse | 45-55 | Gewichtsteile |
| - Meproester | 3,5-4 | Gewichtsteile |
| - Rosenöl | | nach Bedarf |

11. Masse nach einem der Ansprüche 1-6, dadurch gekennzeichnet, dass sie folgende Komponenten enthält:
| | | |
|---|---|---|
| - Glycerin | 52-58 | Gewichtsteile |
| - Weizenstärke | 140-160 | Gewichtsteile |
| - Gelatine-Pulver | 50-55 | Gewichtsteile |
| - Ester eines niederen Diols mit einer Fettsäure | 8-10 | Gewichtsteile |
| - Ricinusöl | 35-40 | Gewichtsteile |
| - Karaya (gequollen in Wasser) | 240-260 | Gewichtsteile |
| - Alaun | 0,05-0,06 | Gewichtsteile |
| - Milchsäure | 8-10 | Gewichtsteile |
| - Meproester | 0,8-1,2 | Gewichtsteile |
| - Rosenöl | | nach Bedarf |
| - Lebensmittelfarbstoff | | nach Bedarf. |

12. Masse nach einem der Ansprüche 1-6, dadurch gekennzeichnet, dass sie folgende Komponenten enthält:
| | | |
|---|---|---|
| - Glycerin | 48-52 | Gewichtsteile |
| - Weizenstärke | 140-160 | Gewichtsteile |
| - Gelatine-Pulver | 51-56 | Gewichtsteile |
| - Ester eines niederen Diols mit einer Fettsäure | 10-14 | Gewichtsteile |
| - Ricinusöl | 35-40 | Gewichtsteile |
| - Alaun | 0,05-0,10 | Gewichtsteile |
| - Milchsäure | 8-10 | Gewichtsteile |
| - Kochsalz | 10-14 | Gewichtsteile |
| - Meproester | 2-3 | Gewichtsteile |
| - Rosenöl | | nach Bedarf |
| - Lebensmittelfarbstoff | | nach Bedarf. |

13. Masse nach einem der Ansprüche 1-12, dadurch gekennzeichnet, dass sie bis zu 60 Gewichtsteile Wasser enthält.

14. Verfahren zur Verflüssigung der Masse nach einem der Ansprüche 1 bis 12, dadurch gekennzeichnet, dass der Masse eine Mischung von Wasser und Ethanol im Verhältnis von etwa 100:1, die Riechstoff und Farbstoff enthält, zugesetzt wird.

15. Verfahren zum Schäumen der Masse nach einem der Ansprüche 1 bis 12, dadurch gekennzeichnet, dass der Masse eine Lösung von Natriumcarbonat und Wasserstoffperoxid zugesetzt wird.

16. Verfahren zur Herstellung einer Form-, Modellier- und Abdeckmasse für Maskenbildnerei und Kosmetik nach einem der Ansprüche 1 bis 12, dadurch gekennzeichnet, dass Wasser und Glycerin gemischt werden und der erhaltenen Mischung unter Rühren bei erhöhter Tempertur Gelatine und anschliessend die übrigen Ingredienzien zugesetzt werden, wobei soviel Wasser verdampft wird, bis die Masse die gewünschte Konsistenz aufweist.

17. Verfahren nach Anspruch 16 zur Herstellung der Masse nach Anspruch 7, dadurch gekennzeichnet, dass 20 ml Wasser und 19 ml 85% Glycerin-Lösung in ein Gefäss gegeben werden, anschliessend mit der erhaltenen Flüssigkeit 21 g Gelatine-Pulver bei einer Temperatur von 80 bis 90°C angerührt werden, bis eine homogene Masse erhalten wird; und dass schliesslich 1 ml einer 25 % Lösung von Meproester zur Konservierung, 3,5 ml Rosenwasser zur Parfümierung und eine ausreichende Menge einer Lösung eines Lebensmittel-Farbstoffes zum Erzielen der gewünschten Farbe zugemischt werden.

## Claims

1. Moulding, modelling and covering material for mask sculpturing and cosmetic use in the form of a fluid to viscous W/O emulsion, characterized in that it is based on glycerin and gelatin, the components of glycerin and gelatin being contained in approximately equal parts by weight.

2. Material according to claim 1, characterized in that it contains one or more additives from the group of pigments, perfumes, emulsifiers and preservatives.

3. Material according to claim 1 or 2, characterized in that it contains 0.1 to 2 ‰ alum.

4. Material according to one of the claims 1 to 3, characterized in that it contains 0.2 to 2.0 % meproester as a preservative.

5. Material according to one of the claims 1 to 4, characterized in that it contains in addition one or more of the following components: ester of a low diol with a fatty acid as an emulsifier, castor oil, urea, wheat starch, gypsum, chewing gum base, karaya, lactic acid and table salt.

6. Material according to claim 5, characterized in that the ester of the lower diol is an ester of glycerin, of ethylene glycol or of 1,2 dihydroxypropane with a natural fatty acid.

7. Material according to one of the claims 1 to 6, characterized in that it contains the following components:
| | | |
|---|---|---|
| - glycerin | 45-50 | parts by weight |
| - gelatin powder | 50-55 | parts by weight |
| - alum | 0.07-0.09 | parts by weight |
| - meproester | 0.5-0.7 | parts by weight |
and, as needed, rose oil as perfume and food colouring.

8. Material according to claim 7, characterized in that it contains in addition the following components:
| | | |
|---|---|---|
| - ester of a lower diol with a fatty acid | 4.5-5.0 | parts by weight |
| - castor oil | 35-40 | parts by weight |
| - urea | 0.04-0.06 | parts by weight |

9. Material according to claim 7, characterized in that it contains in addition the following components:
| | | |
|---|---|---|
| - wheat starch | 140-160 | parts by weight |
| - ester of a lower diol with a fatty acid | 8-10 | parts by weight |
| - castor oil | 34-41 | parts by weight |
| - urea | 0.04-0.06 | parts by weight |

10. Material according to one of the claims 1 to 6, characterized in that it contains the following components:
| | | |
|---|---|---|
| - glycerin | 45-50 | parts by weight |
| - wheat starch | 45-55 | parts by weight |
| - gelatin powder | 50-55 | parts by weight |
| - gypsum | 9-14 | parts by weight |
| - chewing gum base | 45-55 | parts by weight |
| - meproester | 3.5-4 | parts by weight |
| - rose oil | | as needed |

11. Material according to one of the claims 1 to 6, characterized in that it contains the following components:
| | | |
|---|---|---|
| - glycerin | 52-58 | parts by weight |
| - wheat starch | 140-160 | parts by weight |
| - gelatin powder | 50-55 | parts by weight |
| - ester of a lower diol with a fatty acid | 8-10 | parts by weight |
| - castor oil | 35-40 | parts by weight |
| - karaya (soaked in water) | 240-260 | parts by weight |
| - alum | 0.05-0.06 | parts by weight |
| - lactic acid | 8-10 | parts by weight |
| - meproester | 0.8-1.2 | parts by weight |
| - rose oil | | as needed |
| - food colouring | | as needed. |

12. Material according to one of the claims 1 to 6, characterized in that it contains the following components:
| | | |
|---|---|---|
| - glycerin | 48-52 | parts by weight |
| - wheat starch | 140-160 | parts by weight |
| - gelatin powder | 51-56 | parts by weight |
| - ester of a lower diol with a fatty acid | 10-14 | parts by weight |
| - castor oil | 35-40 | parts by weight |
| - alum | 0.05-0.10 | parts by weight |
| - lactic acid | 8-10 | parts by weight |
| - table salt | 10-14 | parts by weight |
| - meproester | 2-3 | parts by weight |
| - rose oil | | as needed |
| - food colouring | | as needed. |

13. Material according to one of the claims 1 to 12, characterized in that it contains up to 60 parts by weight of water.

14. Method of thinning the material according to one of the claims 1 to 12, characterized in that a mixture of water and ethanol in a proportion of about 100:1, which contains perfume and pigment, is added to the material.

15. Method of foaming the material according to one of the claims 1 to 12, characterized in that a solution of sodium carbonate and hydrogen peroxide is added to the material.

16. Method of producing a moulding, modelling and covering material for mask sculpturing and cosmetic use according to one of the claims 1 to 12 characterized in that water and glycerin are mixed, and while stirring at raised temperature gelatin is added to the mixture obtained and subsequently the other ingredients, water being evaporated until the material has the desired consistency.

17. Method according to claim 16 to produce the material according to claim 7, characterized in that 20 ml of water and 19 ml of 85% glycerine solution are put in a vessel, then 21 g of gelatin powder is stirred with the liquid obtained at a temperature of 80 to 90 °C until a homogeneous material is obtained, and in that admixed finally are 1 ml of a 25% solution of meproester as a preservative, 3.5 ml of rose water as a perfume and a sufficient quantity of a solution of food colouring to achieve the desired colour.

## Revendications

1. Matériau de moulage, de modelage et de revêtement pour la confection de masques et pour une utilisation cosmétique sous forme d'une émulsion eau/huile depuis un état fluide jusqu'à un état visqueux, caractérisé en ce qu'il est composé à base de glycérine et de gélatine, les parties des composants de glycérine et de gélatine étant approximativement égales en poids.

2. Matériau selon la revendication 1, caracatérisé en ce qu'il comprend un ou plusieurs additifs du groupe des colorants, des substances odorantes, des émulsifiants et des agents de conservation.

3. Matériau selon la revendication 1 ou la revendication 2, caractérisé en ce qu'il comprend 0,1 à 2‰ d'alun.

4. Matériau selon l'une des revendications 1 à 3, caractérisé en ce qu'il comprend 0,2 à 2,0% de Meproester en tant qu'agent de conservation.

5. Matériau selon l'une des revendications 1 à 4, caractérisé en ce qu'il contient additionnellement l'un ou plusieurs des composants suivants: ester d'un diol bas avec un acide gras comme émulsifiant, huile de ricin, urée, amidon de froment, gypse, masse de base de gomme à mâcher, Karaya, acide lactique et sel de cuisine.

6. Matériau selon la revendication 5, caractérisé en ce que l'ester du diol bas est un ester de glycérine, d'éthylène-glycol ou de 1,2-propanediol avec un acide gras naturel.

7. Matériau selon l'une des revendications 1 à 6, caractérisé en ce qu'il contient les composants suivants:
| | | |
|---|---|---|
| - Glycérine | 45-50 | parties en poids |
| - Gélatine en poudre | 50-55 | parties en poids |
| - Alun | 0,07-0,09 | parties en poids |
| - Meproester | 0,5-0,7 | parties en poids |
de même que, suivant les besoins, de l'huile de rose pour parfumer et des colorants alimentaires.

8. Matériau selon la revendication 7, caractérisé en ce qu'il contient additionnellement les composants suivants:
| | | |
|---|---|---|
| - Ester d'un diol bas avec un acide gras | 4,5-5,0 | parties en poids |
| - Huile de ricin | 35-40 | parties en poids |
| -Urée | 0,04-0,06 | parties en poids |

9. Matériau selon la revendication 7, caractérisé en ce qu'il comprend additionnellement les composants suivants:
| | | |
|---|---|---|
| - Amidon de froment | 140-160 | parties en poids |
| - Ester d'un diol bas avec un acide gras | 8-10 | parties en poids |
| - Huile de ricin | 34-41 | parties en poids |
| -Urée | 0,04-0,06 | parties en poids |

10. Matériau selon l'une des revendications 1 à 6, caractérisé en ce qu'il contient les composants suivants:
| | | |
|---|---|---|
| - Glycérine | 45-50 | parties en poids |
| - Amidon de froment | 45-55 | parties en poids |
| - Gélatine en poudre | 50-55 | parties en poids |
| - Gypse | 9-14 | parties en poids |
| - masse de base de gomme à mâcher | 45-55 | parties en poids |
| - Meproester | 3,5-4 | parties en poids |
| - Huile de rose | | selon les besoins |

11. Matériau selon l'une des revendications 1 à 6, caractérisé en ce qu'il contient les composants suivants:
| | | |
|---|---|---|
| - Glycérine | 52-58 | parties en poids |
| - Amidon de froment | 140-160 | parties en poids |
| - Gélatine en poudre | 50-55 | parties en poids |
| - Ester d'un diol bas avec un acide gras | 8-10 | parties en poids |
| - Huile de ricin | 35-40 | parties en poids |
| - Karaya (gonfflé dans de l'eau) | 240-260 | parties en poids |
| - Alun | 0,05-0,06 | parties en poids |
| - Acide lactique | 8-10 | parties en poids |
| - Meproester | 0,8-1,2 | parties en poids |
| - Huile de rose | | selon les besoins |
| - Colorants alimentaires | | selon les besoins |

12. Matériau selon l'une des revendications 1 à 6, caractérisé en ce qu'il contient les composants suivants:
| | | |
|---|---|---|
| - Glycérine | 48-52 | parties en poids |
| - Amidon de froment | 140-160 | parties en poids |
| - Gélatine en poudre | 51-56 | parties en poids |
| - Ester d'un diol bas avec un acide gras | 10-14 | parties en poids |
| - Huile de ricin | 35-40 | parties en poids |
| - Alun | 0,05-0,10 | parties en poids |
| - Acide lactique | 8-10 | parties en poids |
| - Sel de cuisine | 10-14 | parties en poids |
| - Meproester | 2-3 | parties en poids |
| - Huile de rose | | selon les besoins |
| - Colorants alimentaires | | selon les besoins |

13. Matériau selon l'une des revendications 1 à 12, caractérisé en ce qu'il comprend jusqu'au 60 parties en poids d'eau.

14. Procédé de liquéfaction du matériau selon l'une des revendications 1 à 12, caractérisé en ce qu'on ajoute au matériau un mélange d'eau et d'éthanol dans un rapport d'environ 100:1, qui contient des matières odorantes et des colorants.

15. Procédé pour faire mousser le matériau selon l'une des revendications 1 à 12, caractérisé en ce qu'on ajoute au matériau une solution de carbonate de sodium et d'eau oxygénée.

16. Procédé de préparation d'un matériau de moulage, de modelage et de revêtement pour la confection de masques et pour une utilisation cosmétique selon l'une des revendications 1 à 12, caractérisé en ce qu'on mélange de l'eau et de la glycérine et qu'on ajoute au mélange obtenu en remuant et en augmentant la température, de la gélatine et ensuite les autres ingrédients, en y vaporisant autant d'eau qu'il est nécessaire pour que le matériau présente la consistance désirée.

17. Procédé selon la revendication 16 pour préparer le matériau selon la revendication 7, caractérisé en ce qu'on met dans un récipient 20 ml d'eau et 19 ml d'une solution de glycérine à 85%, à la suite de quoi on mélange au liquide obtenu 21 g de gélatine en poudre en remuant à une température allant de 80 à 90°C jusqu'à l'obtention d'une masse homogène;
et finalement on y mélange 1 ml d'une solution à 25% de meproester pour conserver le matériau, 3,5 ml d'eau de rose pour le parfumer et une quantité suffisante d'une solution d'un colorant alimentaire pour obtenir la couleur désirée.
